# EUROPEAN PATENT SPECIFICATION

(11) **EP 2 675 814 B1**
(45) Date of publication and mention of the grant of the patent: **14.12.2016**
(21) Application number: 12709787.1
(22) Date of filing: 17.02.2012
(51) Int. Cl.: C07D 498/04, A61K 31/407, A61P 31/12, C07K 5/00, A61K 38/03

(54) **HAPROLID AND DERIVATIVES THEREOF AS INHIBITORS OF HCV**
HAPROLID UND DERIVATE DAVON ALS HCV-HEMMER
HAPROLIDE ET SES DÉRIVÉS EN TANT QU'INHIBITEURS DU VHC

(30) Priority: 18.02.2011 EP 11001353
(43) Date of publication of application: 25.12.2013
(73) Proprietor: Helmholtz-Zentrum für Infektionsforschung GmbH, 38124 Braunschweig (DE); Twincore Zentrum für Experimentelle und Klinische Infektionsforschung GmbH, 30625 Hannover (DE)
(72) Inventor: PIETSCHMANN, Thomas, 30559 Hannover (DE); GENTZSCH, Juliane, 30625 Hannover (DE); STEINMETZ, Heinrich, 31139 Hildesheim (DE); KUNZE, Brigitte, 38124 Braunschweig (DE)
(74) Representative: Forstmeyer, Dietmar
(86) International application number: PCT/EP2012/000707
(87) International publication number: WO 2012/110249

(56) References cited:
- WO-A1-2008/101665
- WO-A2-01/81325

## Description

Hepatitis C virus (HCV) causes chronic infection associated with severe liver disease. About 130 million people are chronically infected worldwide. Current treatment with pegylated interferon-α and ribavirin has limited efficacy and is associated with substantial side effects. Although highly potent HCV-specific enzyme inhibitors are in clinical development, drug-resistance and genotype-specific differences in efficacy may limit these novel therapeutics. Therefore, a combination of drugs with distinct mechanisms of action targeting different steps of the viral replication cycle will likely improve viral response rates and therapy success. Furthermore, 25-30% of HIV-infected patients are also positive for HCV and would greatly benefit from an inhibitor that could target both viruses.

It has been an object of the present invention to provide novel antiviral compounds that are preferably inhibitors of hepatitis C virus (HCV).

The present invention relates to one or more compounds of general formula (I) and mixtures thereof: 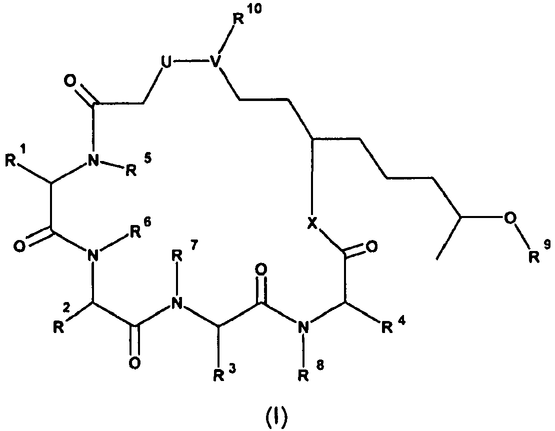 wherein
X is O or NH;
U-V are selected from the following groups: 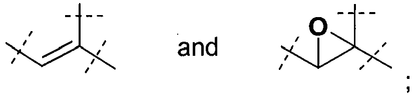
R¹ is a group of formula -CH₂Ar wherein Ar is an optionally substitured aryl or heteroaryl group;
R² and R³ are independently selected from H, CH₃, CH₂OH or the following groups: 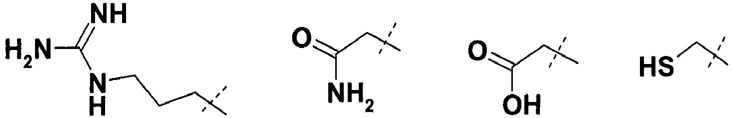 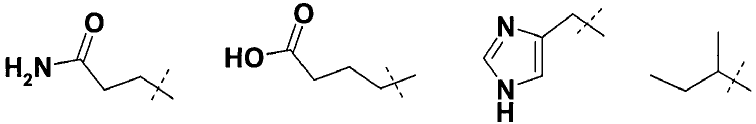 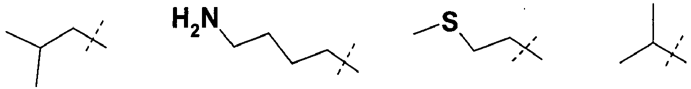 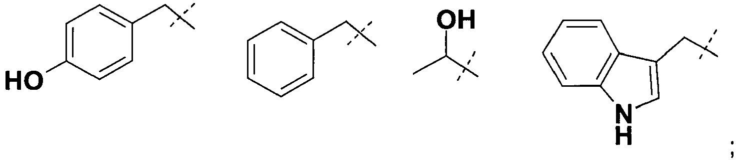
R⁴ and R⁸ together are a group of formula -(CH₂)ₙ- wherein this group may optionally be substituted and n is 2, 3, 4 or 5;
R⁵, R⁶ and R⁷ are independently hydrogen or C₁₋₄ alkyl;
R⁹ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group; and
R¹⁰ is H or methyl;
or a pharmacologically acceptable salt, solvate, or hydrate thereof.

The expression alkyl refers to a saturated, straight-chain or branched hydrocarbon group that contains from 1 to 20 carbon atoms, preferably from 1 to 12 carbon atoms, especially from 1 to 6 carbon atoms, for example a methyl, ethyl, propyl, iso-propyl, n-butyl, iso-butyl, tert-butyl, n-pentyl, n-hexyl, 2,2-dimethylbutyl or n-octyl group.

The expressions alkenyl and alkynyl refer to at least partially unsaturated, straight-chain or branched hydrocarbon groups that contain from 2 to 20 carbon atoms, preferably from 2 to 12 carbon atoms, especially from 2 to 6 carbon atoms, for example an ethenyl, allyl, acetylenyl, propargyl, isoprenyl or hex-2-enyl group. Preferably, alkenyl groups have one or two (especially one) double bond(s) and alkynyl groups have one or two (especially one) triple bond(s).

The expression heteroalkyl refers to an alkyl, alkenyl (e.g. heteroalkenyl) or alkynyl group in which one or more (preferably 1, 2 or 3) carbon atoms have been replaced by an oxygen, nitrogen, phosphorus, boron, selenium, silicon or sulphur atom (preferably oxygen, sulphur or nitrogen). Examples for heteroalkyl groups are alkyloxy, alkyloxyalkyl, alkenyloxyalkyl, alkenyloxy, alkyloxyalkenyl, alkylamino, alkylaminoalkyl, dialkylamino, dialkylaminoalkyl, alkylthio or alkylthioalkyl groups. The expression heteroalkyl furthermore refers to a carboxylic acid or to a group derived from a carboxylic acid such as, for example, acyl, acyloxy, acyloxyalkyl, acylalkyl, alkoxycarbonyl, alkyloxycarbonylalkyl, carboxyalkylamide, alkylcarbonylamino, alkylcarbonylaminoalkyl, alkylaminocarbonyl, alkylaminocarbonylalkyl, or alkoxycarbonyloxy.

Examples of heteroalkyl groups are groups of formulae R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-(preferred are R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}- and R^{a}-N(R^{b})-CO-Y^{a}-,),

R^{a} being a hydrogen atom, a C₁-C₆alkyl, a C₂-C₆alkenyl or a C₂-C₆alkynyl group; R^{b} being a hydrogen atom, a C₁-C₆alkyl, a C₂-C₆alkenyl or a C₂-C₆alkynyl group; R^{c} being a hydrogen atom, a C₁-C₆alkyl, a C₂-C₆alkenyl or a C₂-C₆alkynyl group; R^{d} being a hydrogen atom, a C₁-C₆alkyl, a C₂-C₆alkenyl or a C₂-C₆alkynyl group and Y^{a} being a bond, a C₁-C₆alkylene, a C₂-C₆alkenylene or a C₂-C₆alkynylene group (preferrably, R^{a}, R^{b}, R^{c} and R^{d} are independently H or C₁-C₆ alkyl and Y^{a} is a bond or C₂-C₆alkylene),
each heteroalkyl group containing at least one carbon atom and it being possible for one or more hydrogen atoms to have been replaced by fluorine or chlorine atoms. Specific examples of heteroalkyl groups are methoxy, trifluoromethoxy, ethoxy, n-propyloxy, isopropyloxy, *tert*-butyloxy, methoxymethyl, ethoxymethyl, methoxyethyl, methylamino, ethylamino, dimethylamino, diethylamino, isopropylethylamino, methylaminomethyl, ethylaminomethyl, diisopropylaminoethyl, enol ether, dimethylaminomethyl, dimethylaminoethyl, acetyl, propionyl, butyryloxy, acetyloxy, methoxycarbonyl, ethoxycarbonyl, N-ethyl-N-methylcarbamoyl and N-methylcarbamoyl. Further examples of heteroalkyl groups are nitrile, isonitrile, cyanate, thiocyanate, isocyanate, isothiocyanate and alkylnitrile groups.

Furthermore, the terms alkyl, alkenyl, alkynyl and heteroalkyl refer to groups in which one or more hydrogen atoms have been replaced by a halogen atom (preferably F or Cl) such as, for example, a 2,2,2-trichloroethyl or a trifluoromethyl or a trifluoromethyloxy group.

The expression cycloalkyl refers to a saturated or partially unsaturated (for example cycloalkenyl), cyclic group that contains one or more rings (preferably 1 or 2), containing from 3 to 50 carbon atoms, preferably 3 to 14, further preferably from 3 to 10 (especially 3, 4, 5, 6 or 7) carbon atoms. The expression cycloalkyl refers furthermore to groups in which one or more hydrogen atoms have been replaced by fluorine, chlorine, bromine or iodine atoms or by OH, =O, SH, =S, NH₂, =NH or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetralin, cyclopentylcyclohexyl, fluorocyclohexyl or cyclohex-2-enyl group.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulphur atom (preferably oxygen, sulphur or nitrogen). A heterocycloalkyl group has preferably 1 or 2 ring(s) containing from 3 to 10 (especially 3, 4, 5, 6 or 7) ring atoms. The expression heterocycloalkyl refers furthermore to groups in which one or more hydrogen atoms have been replaced by fluorine, chlorine, bromine or iodine atoms or by OH, =O, SH, =S, NH₂, =NH or NO₂ groups. Examples are a piperidyl, piperazinyl, morpholinyl, urotropinyl, pyrrolidinyl, tetrahydrothiophenyl, tetrahydropyranyl, tetrahydrofuryl or 2-pyrazolinyl group and also lactams, lactones, cyclic imides and cyclic anhydrides.

The expression alkylcycloalkyl refers to groups containing both cycloalkyl and also alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings containing from 3 to 10 (especially 3, 4, 5, 6 or 7) carbon atoms, and one or two alkyl, alkenyl or alkynyl groups having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulphur atom (preferably oxygen, sulphur or nitrogen). A heteroalkylcycloalkyl group preferably contains 1 or 2 ring systems having from 3 to 10 (especially 3, 4, 5, 6 or 7) ring atoms, and one or two alkyl, alkenyl, alkynyl or heteroalkyl groups having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that has one or more rings containing from 6 to 50 carbon atoms, preferably 6 to 14, further preferably from 6 to 10 (especially 6) carbon atoms. The expression aryl refers furthermore to groups in which one or more hydrogen atoms have been replaced by fluorine, chlorine, bromine or iodine atoms or by OH, SH, NH₂ or NO₂ groups. Examples are a phenyl, naphthyl, biphenyl, 2-fluorophenyl, anilinyl, 3-nitrophenyl or 4-hydroxyphenyl group.

The expression heteroaryl refers to an aromatic group that has one or more rings containing from 5 to 50 ring atoms, preferably 5 to 14, further preferably from 5 to 10 (especially 5 or 6) ring atoms, and contains one or more (preferably 1, 2, 3 or 4) oxygen, nitrogen, phosphorus or sulphur ring atoms (preferably O, S or N). The expression heteroaryl refers furthermore to groups in which one or more hydrogen atoms have been replaced by fluorine, chlorine, bromine or iodine atoms or by OH, SH, NH₂ or NO₂ groups. Examples are 4-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1H-indene, tetralin, dihydronaphthalene, indanone, phenylcyclopentyl, cumene, cyclohexylphenyl, fluorene and indan. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to an aralkyl group as defined above in which one or more (preferably 1, 2, 3 or 4) carbon atoms have been replaced by an oxygen, nitrogen, silicon, selenium, phosphorus, boron or sulphur atom (preferably oxygen, sulphur or nitrogen), that is to say to groups containing both aryl or heteroaryl and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced by oxygen, sulphur or nitrogen atoms.

Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl and heteroarylheteroalkylheterocycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl also refer to groups in which one or more hydrogen atoms of such groups have been replaced by fluorine, chlorine, bromine or iodine atoms or by OH, =O, SH, =S, NH₂, =NH or NO₂ groups.

Furthermore, preferably all alkyl, alkenyl, alkynyl, heteroalkyl, cycloalkyl, heterocycloalkyl, alkylcycloalkyl, heteroalkylcycloalkyl, aryl, heteroaryl, aralkyl and heteroaralkyl groups as defined herein may be optionally substituted.

The expression "optionally substituted" refers to groups in which one or more hydrogen atoms optionally have been replaced by fluorine, chlorine, bromine or iodine atoms or by OH, =O, SH, =S, NH₂, =NH or NO₂ groups. This expression refers furthermore to groups that are optionally substituted by one or more (e.g. 1, 2 or 3) unsubstituted C₁-C₆alkyl, C₂-C₆alkenyl, C₂-C₆alkynyl, C₁-C₆heteroalkyl, C₃-C₁₀cycloalkyl, C₂-C₉heterocycloalkyl, C₆-C₁₀aryl, C₁-C₉heteroaryl, C₇-C₁₂aralkyl or C₂-C₁₁heteroaralkyl groups.

Owing to their substitution, compounds of formula (I) may contain one or more centres of chirality. The present invention therefore includes both all pure enantiomers and all pure diastereoisomers and also mixtures thereof in any mixing ratio. The present invention moreover also includes all cis/trans-isomers of the compounds of the general formula (I) and also mixtures thereof. The present invention moreover includes all tautomeric forms of the compounds of formula (I).

Preferably, X is O.

Further preferably, R⁹ is methyl.

Further preferably, R¹⁰ is methyl.

Moreover preferably, R⁵, R⁶ and R⁷ are independently hydrogen or methyl.

Further preferably, R⁵ is hydrogen.

Further preferably, R⁶ is methyl.

Further preferably, R⁷ is methyl.

R⁴ and R⁸ together are a group of formula -(CH₂)ₙ- wherein this group may optionally be substituted (e.g. by a hydroxy group) and n is 2, 3, 4 or 5.

Especially preferably, R⁴ and R⁸ together are a group of formula -(CH₂)₃-.

Further preferably, R² is hydrogen.

Especially preferably, R³ is a group of formula -CH₂CH(CH₃)₂.

Further preferably, R¹ is a group of formula -CH₂Ar wherein Ar is a phenyl group.

Further preferred are compounds of formula (II): 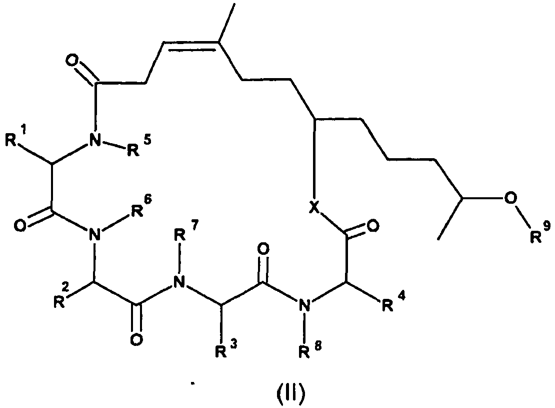 wherein R¹, R², R³, R⁴, R⁵, R⁶, R⁷ R⁸, R⁹ and X are as defined above, or a pharmacologically acceptable salt, solvate, or hydrate thereof.

Especially preferred, the compound of formula (I) or (II) has the following structure (Haprolid):

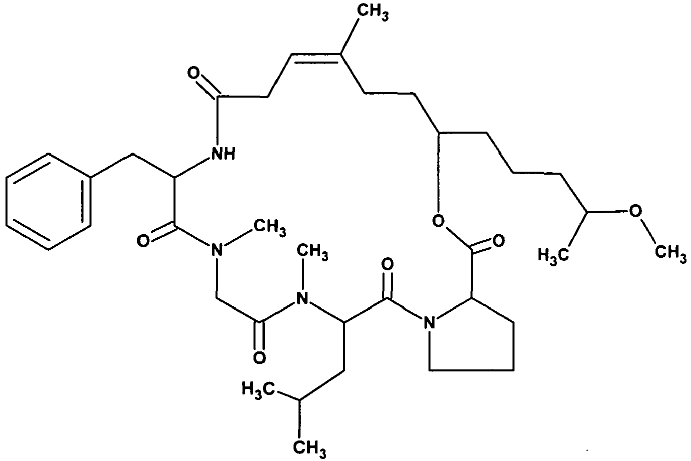

The therapeutic use of compounds of formula (I) or (II), their pharmacologically acceptable salts or solvates and hydrates and also formulations and pharmaceutical compositions also lie within the scope of the present invention, especially, the use of compounds of formula (I) or (II), their pharmacologically acceptable salts or solvates and hydrates and also formulations and pharmaceutical compositions as antiviral agents, especially for the treatment of hepatitis C as well as their use for the preparation of medicaments for the treatment of viral infections, especially for the treatment of hepatitis C. Furthermore, the present invention relates to compounds and/or pharmaceutical compositions as described herein for use in the treatment of viral infections, especially for use in the treatment of hepatitis C.

The pharmaceutical compositions according to the present invention comprise at least one compound of formula (I) or (II) as active ingredient and, optionally, carrier substances and/or adjuvants. Further, these pharmaceutically compositions may comprise other antiviral ingredients.

Examples of pharmacologically acceptable salts of the compounds of formula (I) or (II) are salts of physiologically acceptable mineral acids, such as hydrochloric acid, sulphuric acid and phosphoric acid, or salts of organic acids, such as methanesulphonic acid, p-toluenesulphonic acid, lactic acid, acetic acid, trifluoroacetic acid, citric acid, succinic acid, fumaric acid, maleic acid and salicylic acid. Further examples of pharmacologically acceptable salts of the compounds of formula (I) or (II) are alkali metal and alkaline earth metal salts such as, for example, sodium, potassium, lithium, calcium or magnesium salts, ammonium salts or salts of organic bases such as, for example, methylamine, dimethylamine, triethylamine, piperidine, ethylenediamine, lysine, choline hydroxide, meglumine, morpholine or arginine salts. Compounds of formula (I) or (II) may be solvated, especially hydrated. The hydration may take place, for example, during the preparation process or as a consequence of the hygroscopic nature of the initially anhydrous compounds of formula (I) or (II). When the compounds of formula (I) or (II) comprise asymmetric C-atoms, they may be present either in the form of achiral compounds, diastereoisomeric mixtures, mixtures of enantiomers or in the form of optically pure compounds.

The pro-drugs to which the present invention also relates comprise a compound of formula (I) or (II) and at least one pharmacologically acceptable protecting group which will be removed under physiological conditions, such as, for example, an alkoxy-, aralkyloxy-, acyl- or acyloxy group, such as, for example, an ethoxy, benzyloxy, acetyl or acetyloxy group.

The present invention relates also to the use of those active ingredients in the preparation of medicaments (especially antiviral drugs). In general, compounds of formula (I) or (II) are administered either individually, or in combination with any other desired therapeutic agent, using the known and acceptable methods. Such therapeutically useful agents may be administered, for example, by one of the following routes: orally, for example in the form of dragées, coated tablets, pills, semisolid substances, soft or hard capsules, solutions, emulsions or suspensions; parenterally, for example in the form of an injectable solution; rectally in the form of suppositories; by inhalation, for example in the form of a powder formulation or a spray; transdermally or intranasally. For the preparation of such tablets, pills, semisolid substances, coated tablets, dragées and hard gelatine capsules, the therapeutically usable product may be mixed with pharmacologically inert, inorganic or organic pharmaceutical carrier substances, for example with lactose, sucrose, glucose, gelatine, malt, silica gel, starch or derivatives thereof, talcum, stearic acid or salts thereof, skimmed milk powder, and the like. For the preparation of soft capsules, pharmaceutical carrier substances such as, for example, vegetable oils, petroleum, animal or synthetic oils, wax, fat and polyols may be used. For the preparation of liquid solutions and syrups, pharmaceutical carrier substances such as, for example, water, alcohols, aqueous saline solution, aqueous dextrose, polyols, glycerol, vegetable oils, petroleum and animal or synthetic oils may be used. For suppositories, pharmaceutical carrier substances such as, for example, vegetable oils, petroleum, animal or synthetic oils, wax, fat and polyols may be used. For aerosol formulations, compressed gases that are suitable for this purpose, such as, for example, oxygen, nitrogen and carbon dioxide may be used. The pharmaceutically acceptable agents may also comprise additives for preserving and stabilising, emulsifiers, sweeteners, flavourings, salts for altering the osmotic pressure, buffers, encapsulation additives and antioxidants.

Combinations with other therapeutic agents may comprise other antiviral agents, especially other inhibitors of hepatitis C Virus (HCV).

For the prevention and/or treatment of viral infections such as hepatitis C, the dose of the biologically active compound according to the invention may vary within wide limits and may be adjusted to individual requirements. Generally, a dose of from 10 mg to 4000 mg per day is suitable, a preferred dose being from 50 to 3000 mg per day. In suitable cases, the dose may also be below or above the stated values. The daily dose may be administered as a single dose or in a plurality of doses. A typical individual dose contains approximately 50 mg, 100 mg, 250 mg, 500 mg, 1 g or 2 g of the active ingredient.

The invention also relates to a method of preventing or treating a viral infection, particularly a HCV infection, wherein the method comprises administration of at least one compound according to the invention, or a pharmacologically acceptable salt, solvate, or hydrate thereof, or a pharmaceutical composition according to the invention.

The method of preventing or treating a HCV infection according to the invention may, moreover, be characterized in that the compound according to the invention, or a pharmacologically acceptable salt, solvate, or hydrate thereof, or a pharmaceutical composition according to the invention is intended to be administered by oral route, by aerosol route or by injection.

It is especially preferred to combine the preferred embodiments of the present invention in any possible manner.

### EXAMPLE

### Isolation of Haprolid:

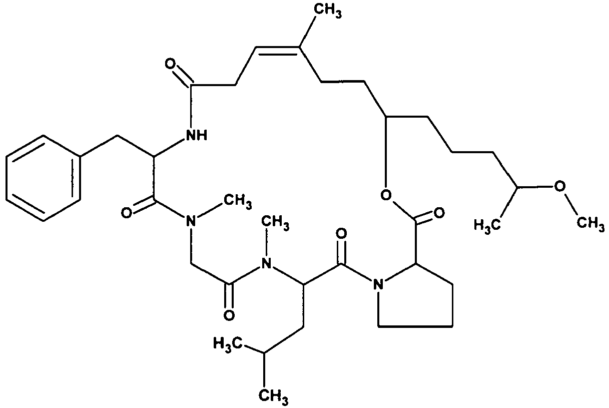

A fermentation (90L) of *Byssovorax cruenta,* strain Har1 (The strain has been deposited at the German Collection of Microorganisms and Cell Cultures (DSMZ) Braunschweig, Germany under the accession number DSM 14567), was performed in presence of 0.9 L Amberlite XAD 16. At the end of the fermentation, the resin was harvested by filtration with a process filter (210 µm mesh), washed with water to remove adherent cells, and extracted with 5 L of acetone to yield raw haprolid. After evaporation of the acetone, the residual water layer was extracted two times with ethyl acetate. The organic layer was dried, filtrated and concentrated in vacuo to give a crude extract (yield 7.9 g).

Initial separation was achieved by silica gel column chromatography (130 g of silica gel Merck 60 15-40 µm) with the eluents: 1. ethyl acetate/n-heptane 2:1 (0.9 L); 2. ethyl acetate (0.9 L); 3. ethyl acetate/methanol 8:2 (0.7 L); 4. ethyl acetate/methanol 1:1 (0.6 L). Based on TLC analyses the chromatography gave 4 fractions. The third fraction (0.73 g) was further separated [column 30×480 mm, Kronlab ODS-AQ 120 16 µm, solvent: acetonitrile/water 55:45, flow rate: 18 mL/min, UV detection 206 nm]. After evaporation of the organic solvent from the haprolid containing fraction, the remaining water was extracted with ethyl acetate to yield 116 mg pure haprolid.

Physico-chemical characterisation of Haprolid:
Formula: C₃₈H₅₈N₄O₇
Molecular weight: 682.9 g/mol
HR ESI MS: [M+H]⁺ calcd.: 683.4378 found: 683.4383
IR (KBr): v = 2957, 2925, 2870, 1738, 1650, 1452, 1193 cm⁻¹.
UV (MeOH): λₘₐₓ (Ig ε) = 205 (4.49), 220 (sh) nm.
TLC (silica gel): solvent dichloromethane:methanol 9:1, *R*_{f} = 0.75
HPLC: column 2×125 mm, Nucleodur Gravity 120 5µm C18 (Macherey Nagel),
   solvent A: 95/5 water/acetonitrile + 5 mM NH₄Ac, pH: 5.5
   solvent B: 5/95 water/acetonitrile + 5 mM NH₄Ac, pH: 5.5
   55% B to 60% B in 10 min, 60% B isocratic
   flow 0.3 mL/min,
   detection UV 200-400 nm
   *R*ₜ = 5.8 min.

**Table 1: NMR-Data of Haprolid in DMSO-d₆**

| phenylalanine proton | δ (ppm) | m | J [Hz] | carbon | δ (ppm) |
|---|---|---|---|---|---|
| | | | | C-1 | 169.9 |
| 2-H | 4.87 | ddd | 9.6, 9.6, 5.4 | C-2 | 49.6 |
| 3-H_{b} | 3.04 | dd | 13.8, 5.4 | C-3 | 37.5 |
| 3-Hₐ | 2.75 | dd | 13.9,4.2 | | |
| | | | | C-4 | 138.1 |
| 5,6-H | 7.2 | m | | C-5,6 | 129.3 |
| 7,8-H | 7.24 | m | | C-7,8 | 127.4 |
| 9-H | 7.19 | m | | C-9 | 125.4 |
| 2-NH | 8.28 | d | 10.0 | | |

| sarkosine | | | | | |
|---|---|---|---|---|---|
| | | | | C-1 | 168.3 |
| 2-H_{b} | 4.75 | d | 17.6 | C-2 | 50.2 |
| 2-Hₐ | 3.83 | d | 17.6 | | |
| 2-NH₃ | 2.81 | s | | N-CH₃ | 34.7 |

| *N*-methyl-leucine | | | | | |
|---|---|---|---|---|---|
| | | | | C-1 | 169.6 |
| 2-H | 5.32 | dd | 11.4, 3.8 | C-2 | 51.2 |
| 3-H_{b} | 1.74-1.66 | m | | C-3 | 36.5 |
| 3-Hₐ | 1.39-1.32 | m | | | |
| 4-H | 1.48-1.38 | m | | C-4 | 24.2 |
| 5-H₃ | 0.86 | d | 6.5 | C-5 | 20.7 |
| 6-H₃ | 0.91 | d | 6.5 | C-6 | 22.7 |
| 2-NH₃ | 2.84 | s | | N-CH₃ | 29.1 |

| proline | | | | | |
|---|---|---|---|---|---|
| 2-H | 4.23 | d | 8.8, 2.8 | C-1 | 171.9 |
| 3 - H_{b} | 2.16-2.11 | m | | C-2 | 58.4 |
| 3 - Hₐ | 1.80-1.76 | m | | C-3 | 28.4 |
| 4 - H_{b} | 2.02-1.96 | m | | C-4 | 24.0 |
| 4 - Hₐ | 2.02-1.96 | m | | C-5 | 46.0 |
| 5 - H_{b} | 3.64-3.59 | m | | | |
| 5 - Hₐ | 3.55 | ddd | 9.0, 9.0, 7.6 | | |

| substituted unsaturated carboxylic acid | | | | | |
|---|---|---|---|---|---|
| proton | δ (ppm) | m | J [Hz] | carbon | δ (ppm) |
| | | | | C-1 | 170.1 |
| 2-H_{b} | 2.64 | dd | 14.1, 11.1 | C-2 | 35.2 |
| 2-Hₐ | 2.29 | dbr. | 14.3 | | |
| 3-H | 4.52 | dbr. | 9.7 | C-3 | 121.1 |
| | | | | C-4 | 134.2 |
| 5-H_{b} | 2.20-2.14 | m | | C-5 | 26.4 |
| 5-Hₐ | 1.73-1.69 | m | | | |
| 6-H_{b} | 1.64-1.56 | m | | C-6 | 30.9 |
| 6-Hₐ | 1.64-1.56 | m | | | |
| 7-H | 4.62 | m | | C-7 | 72.2 |
| 8-H_{b} | 1.46-1.40 | m | | C-8 | 34.5 |
| 8-Hₐ | 1.46-1.40 | m | | | |
| 9-H_{b} | 1.40-1.20 | m | | C-9 | 20.2 |
| 9-Hₐ | 1.40-1.20 | m | | | |
| 10-H_{b} | 1.40-1.29 | m | | C-10 | 35.1 |
| 10-Hₐ | 1.40-1.29 | m | | | |
| 11-H | 3.23 | m | | C-11 | 75.4 |
| 4-Me | 1.52 | s | | C-4 Me | 22.2 |
| 11-Me | 1.03 | d | 6.2 | C-11 Me | 18.5 |
| 11-OMe | 3.19 | s | | C-11 OMe | 54.8 |

### Cell-based assay for screening the complete HCV replication cycle (4):

To identify novel bioactive compounds with HCV-specific antiviral activity, a robust and sensitive cell based assay based on a cell line derived from Huh-7 human hepatocarcinoma cells has been developed. The cells used in this assay originate from Huh7-Lunet cells that are highly permissive to HCV RNA replication (1), but which express only limited quantities of CD81, an essential HCV entry factor (2). Robust expression of human CD81 was established using lentiviral gene transfer thus rendering these cells also highly susceptible to HCV cell entry (3). Further, a lentiviral vector has been employed to introduce a constitutively expressed transgene for a secreted gaussia luciferase (G-Luc) from the marine copepod *Gaussia princeps.* The resulting cell line was designated Huh7-Lunet-hCD81-Gluc and is highly permissive for HCV propagation. In addition it expresses a simple biomarker (G-Luc) which reflects both cell number and cell viability and that can be monitored non-invasively by sampling the culture fluid. To quantify HCV replication in these cells, the highly efficient HCV reporter virus Luc-Jc1 has been utilized which expresses the firefly luciferase derived from *Photinus pyralis* (2). As a consequence, cell viability and HCV replication can be measured simultaneously using a dual luciferase assay determining G-Luc and F-Luc activities in culture fluids and cell lysates, respectively.

To permit simple detection of influences on RNA replication, virus production and also cell entry, a screening protocol has been developed that encompasses the entire HCV replication cycle and that is based on two measurements of HCV-dependent F-Luc activity: First, Huh7-Lunet-hCD81-Gluc cells are transfected with Luc-Jc1 RNA and seeded into culture plates. Four hours later, individual compounds are added to the culture fluid and co-cultured with the HCV-replicating cells throughout the entire assay. A first reading of F-Luc activity is conducted 48 h post transfection and reflects HCV RNA translation and replication efficiency in the presence of compounds since at this time point secondary rounds of infection do not significantly contribute to the HCV-specific F-Luc signal (2). In parallel, culture fluid of these cells containing infectious reporter viruses produced at this time point as well as the compounds are transferred to naive Huh7-Lunet-hCD81-Gluc cells. Another 48 h later, both G-Luc and F-Luc activities produced in the inoculated cells are determined. These readings reflect cell viability and the efficiency of HCV RNA replication, virus production and cell entry, thus encompassing at least one entire replication cycle of HCV in the presence of compounds.

### References:

1. Friebe, P., Boudet, J., Simorre, J. P., and Bartenschlager, R. 2005. Kissing-loop interaction in the 3' end oft he hepatitis C virus genome essential for RNA replication. J. Virol. 79: 380-392.
2. Koutsoudakis, G., Kaul, A., Steinmann, E., Kallis, S. Lohmann, V., Pietschmann, T., and Bartenschlager, R. 2006. Characterization of the early steps of hepatitis C virus infection by using luciferase reporter viruses. J. Virol. 80: 5308-5320.
3. Bitzegeio, J., Bankwitz, D., Hueging, K., Haid, S., Brohm, C., Zeisel, M.B., Herrmann, E., Iken, M., Ott, M., Baumert, T.F., and Pietschmann, T. 2010. Adaptation of hepatitis C virus to mouse CD81 permits infection of mouse cells in the absence of human entry factors. PLoS Pathog. 6:e1000978.
4. Gentzsch, J., Hinkelmann, B., Kaderali, L., Irschik, H., Jansen, R., Sasse, F., Frank, R., and Pietschmann, T. 2011. Hepatitis C virus complete life cycle screen for identification of small molecules with pro- or antiviral activity. Antiviral Res. 89 (2): 136-48.

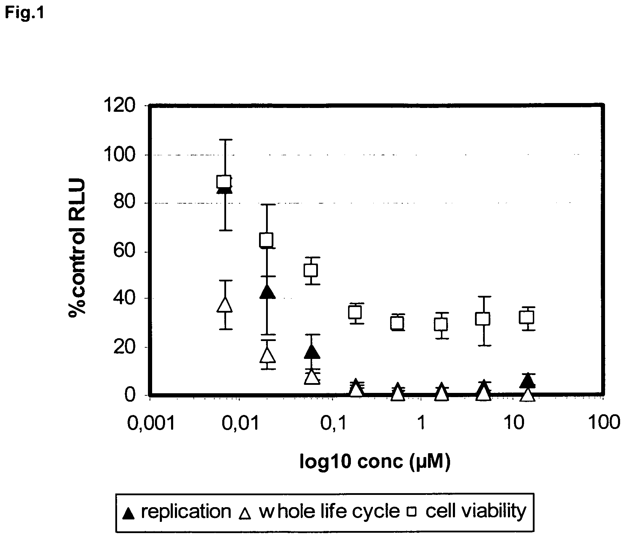

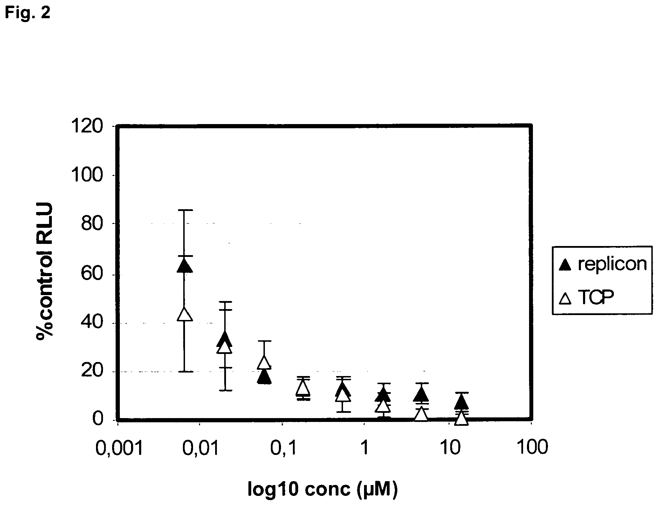

Haprolid has been identified as a potent inhibitor of HCV. Experiments confirmed its antiviral activity at nanomolar concentrations (EC50 < 6nM) with toxicity in low micromolar ranges (TLC50 ∼ 0.1 µM) (Fig. 1). The compound displayed a moderate influence on HCV RNA replication (5-fold reduction at non-toxic concentrations, Fig. 2) as shown with the subgenomic HCV replicon system, and a similar influence when analyzed with HCV_{TCP}. Employing HCV_{TCP}, influences on HCV RNA replication and virus entry are quantified. Since no increased susceptibility of HCV_{TCP} to the drug compared to subgenomic replicons has been observed, these data indicate that the drug primarily affects HCV RNA replication but not the HCV cell entry steps (Fig. 2).

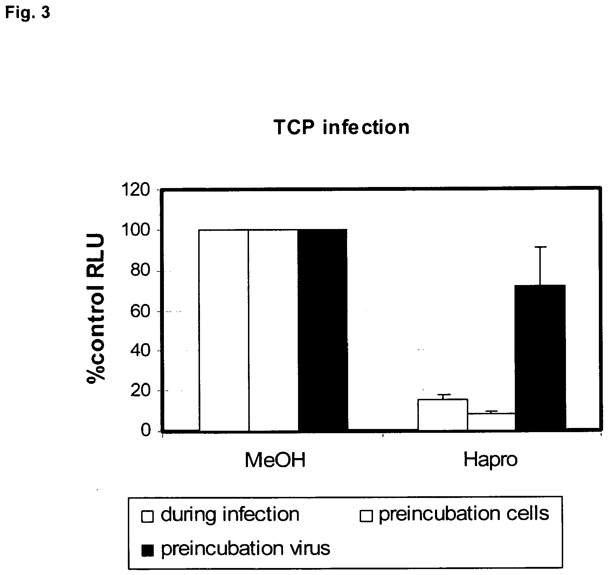

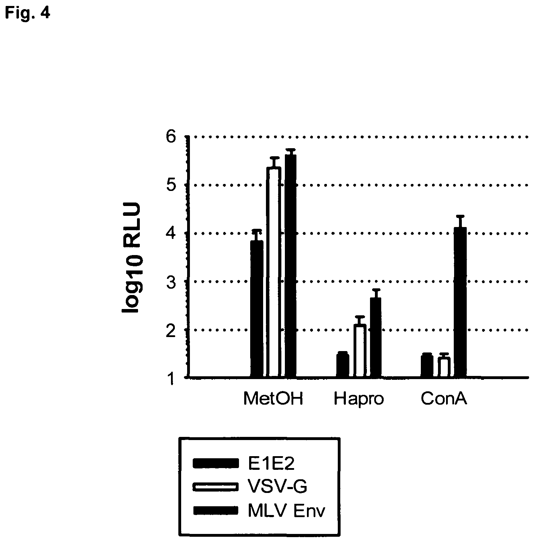

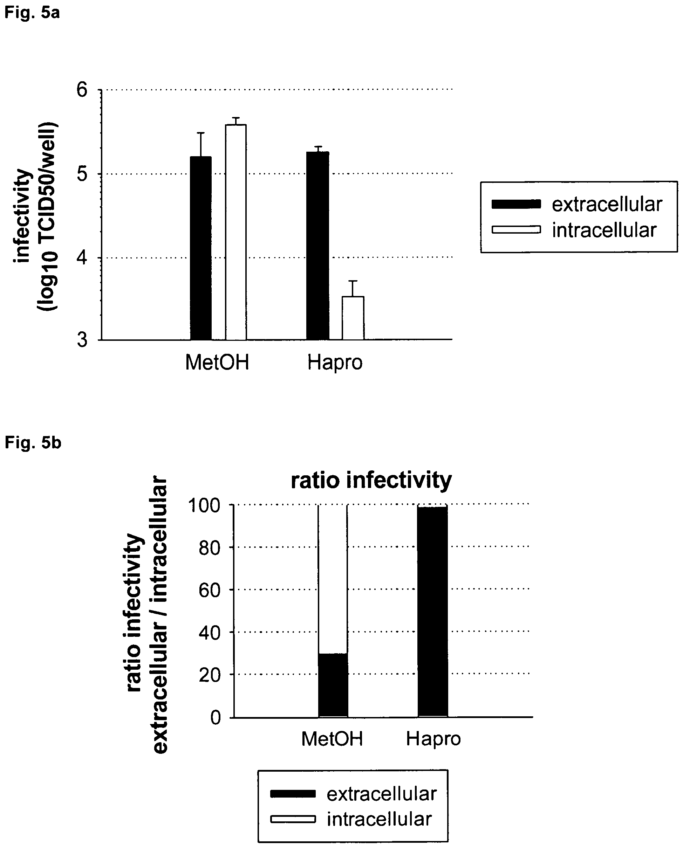

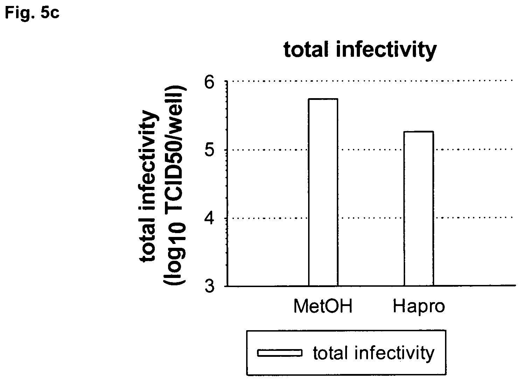

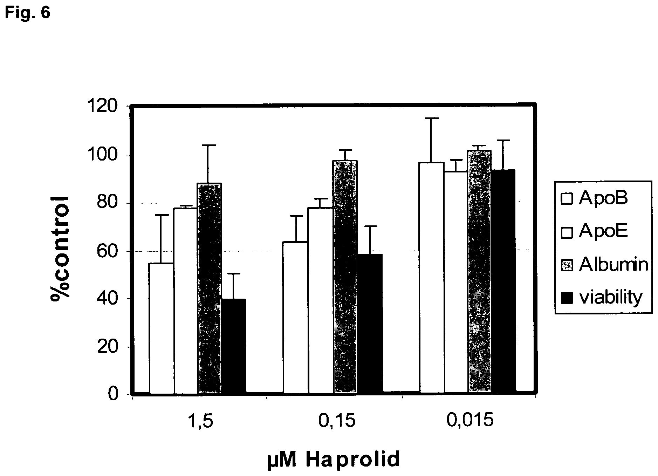

Adding the compound to retroviral particles pseudotyped with the glycoproteins of either HCV, VSV (vesicular stomatitis virus) or MLV (murine leukemia virus), another method of testing for viral entry, resulted in a strong decrease of infectivity (100- to 1000-fold, Fig. 4). These results indicate that Haprolid interferes with retroviral cell entry steps in a viral glycoprotein-independent fashion possible blocking steps like e.g. trafficking of retroviral cores, reverse transcription, nuclear import, or integration. Moreover, an influence of the compound on the steps leading to assembly and release of infectious HCV particles has been shown. Total production of infectious virus particles was reduced about 3-fold (Fig. 5c). Addition of the compound led to a 100-fold reduction of intracellular virus titer (Fig. 5a), indicating that processes determining export of virus particles from infected cells are modulated by the drug. However, this was not caused by an influence of the drug on lipoprotein secretion (ApoB/E, Fig. 6), on which HCV secretion strongly depends, nor a general influence on the secretory capacity of the cell as shown for albumin (Fig. 6). Finally, preincubation experiments utilizing the HCV_{TCP} system revealed an influence of the compound on the cell rather than on viral component since only preincubation of the cells, but not of the virus resulted in a comparable effect as addition of compound during infection (Fig. 3).

In conclusion, a novel, potent HCV inhibitor has been identified which affects assembly and release of particles as well as HCV replication. Additionally, it interferes with early steps of the retroviral life cylce indicating a broader scope of antiviral activity. This inhibitor influences the host cell rather than the virus itself which could be beneficial in the prevention of resistance mutations which often occur during HCV infection.
Figures 1 to 6 show a summary of the influence (effects) of Haprolid on HCV:
   Figure 1 shows the results of a whole life cycle assay.
   Figure 2 shows the results of a replicon assay (influence on replication) vs. TCP assay (replication+entry).
   Figure 3 shows the results of a preincubation experiment in the context of HCV TCP infection.
   Figure 4 shows the results of a pseudoparticle infection assay.
   Figures 5a-c show the influence on assembly and/or release.
   Figure 6 shows that there is no influence on secretion of lipoproteins or albumin.

## Claims

1. Compound of general formula (I): 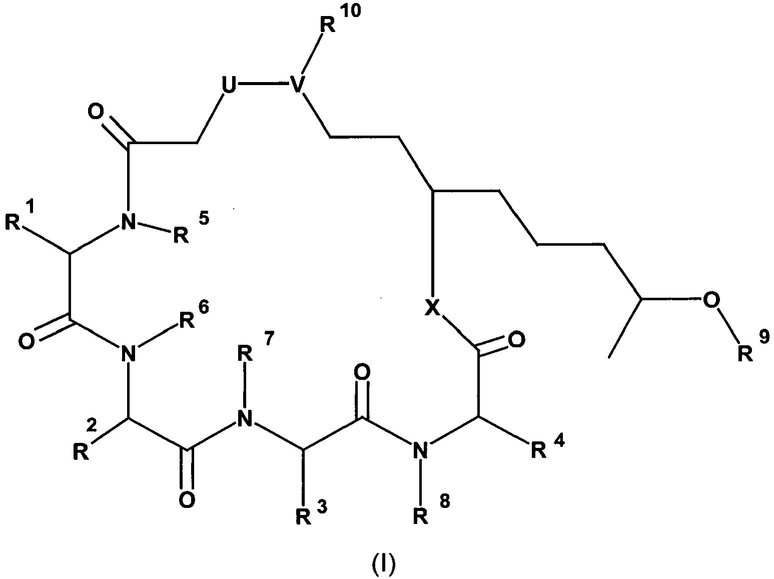 wherein
X is O or NH;
U-V are selected from the following groups: 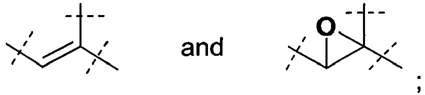
R¹ is a group of formula -CH₂Ar wherein Ar is an optionally substitured aryl or heteroaryl group; 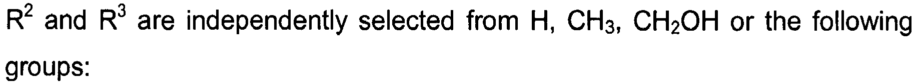 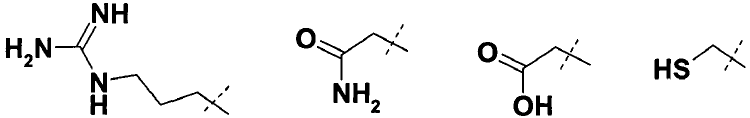 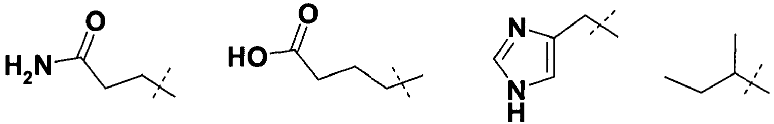 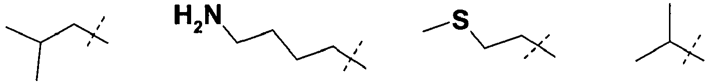 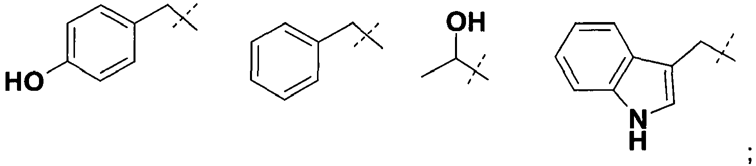
R⁴ and R⁸ together are a group of formula -(CH₂)ₙ- wherein this group may optionally be substituted and n is 2, 3, 4 or 5;
R⁵, R⁶ and R⁷ are independently hydrogen or C₁₋₄ alkyl;
R⁹ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group; and
R¹⁰ is H or methyl;
or a pharmacologically acceptable salt, solvate, or hydrate thereof;
wherein
the expression alkyl refers to a saturated, straight-chain or branched hydrocarbon group that contains from 1 to 6 carbon atoms;
the expressions alkenyl and alkynyl refer to at least partially unsaturated, straight-chain or branched hydrocarbon groups that contain from 2 to 6 carbon atoms;
the expression heteroalkyl refers to an alkyl, alkenyl or alkynyl group in which one or more carbon atoms have been replaced by an oxygen, nitrogen or sulphur atom;
the terms alkyl, alkenyl, alkynyl and heteroalkyl refer furthermore to groups in which one or more hydrogen atoms have been replaced by a halogen atom;
the expression cycloalkyl refers to a saturated or partially unsaturated, cyclic group that contains one or two rings, containing from 3 to 10 carbon atoms; the expression cycloalkyl refers furthermore to groups in which one or more hydrogen atoms have been replaced by fluorine, chlorine, bromine or iodine atoms or by OH, =O, SH, =S, NH₂, =NH or NO₂ groups;
the expression heterocycloalkyl refers to a cycloalkyl group as defined above in which one or more ring carbon atoms have been replaced by an oxygen, nitrogen or sulphur atom;
the expression alkylcycloalkyl refers to groups containing both cycloalkyl and also alkyl, alkenyl or alkynyl groups in accordance with the above definitions;
the expression heteroalkylcycloalkyl refers to alkylcycloalkyl groups as defined above in which one or more carbon atoms have been replaced by an oxygen, nitrogen or sulphur atom;
the expression aryl refers to an aromatic group that has one or more rings containing from 6 to 10 carbon atoms; the expression aryl refers furthermore to groups in which one or more hydrogen atoms have been replaced by fluorine, chlorine, bromine or iodine atoms or by OH, SH, NH₂ or NO₂ groups;
the expression heteroaryl refers to an aromatic group that has one or more rings containing from 5 to 10 ring atoms, and contains one or more oxygen, nitrogen or sulphur ring atoms; the expression heteroaryl refers furthermore to groups in which one or more hydrogen atoms have been replaced by fluorine, chlorine, bromine or iodine atoms or by OH, SH, NH₂ or NO₂ groups;
the expression aralkyl refers to groups containing both aryl and also alkyl, alkenyl, alkynyl and/or cycloalkyl groups in accordance with the above definitions;
the expression heteroaralkyl refers to an aralkyl group as defined above in which one or more carbon atoms have been replaced by an oxygen, nitrogen or sulphur atom; and
the expression "optionally substituted" refers to groups in which one or more hydrogen atoms optionally have been replaced by fluorine, chlorine, bromine or iodine atoms or by OH, =O, SH, =S, NH₂, =NH or NO₂ groups and wherein this expression refers furthermore to groups that are optionally substituted by one or more unsubstituted C₁-C₆alkyl, C₂-C₆alkenyl, C₂-C₆alkynyl, C₁₋C₆heteroalkyl, C₃-C₁₀cycloalkyl, C₂-C₉heterocycloalkyl, C₆-C₁₀aryl, C₁-C₉heteroaryl, C₇-C₁₂aralkyl or C₂-C₁₁heteroaralkyl groups.

2. Compound according to claim 1, having the following general formula (II): 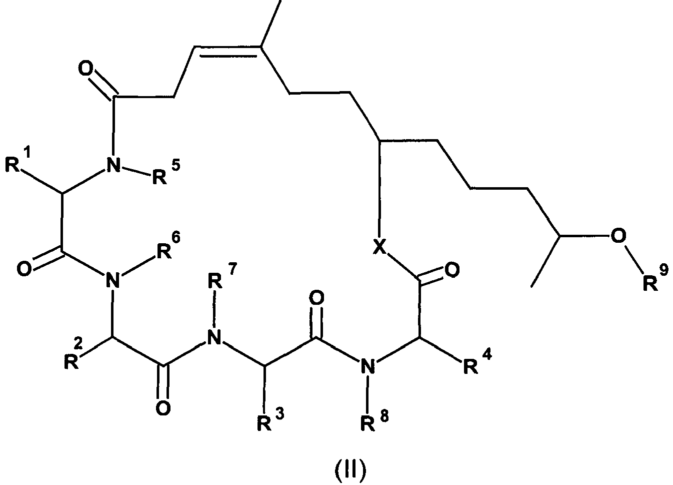 wherein R¹, R², R³, R⁴, R⁵, R⁶, R⁷ R⁸, R⁹ and X are defined as in Claim 1, or a pharmacologically acceptable salt, solvate, or hydrate thereof.

3. Compound according to claim 1 or 2, wherein X is O, or a pharmacologically acceptable salt, solvate, or hydrate thereof.

4. Compound according to any one of claims 1 to 3, wherein R⁹ is methyl, or a pharmacologically acceptable salt, solvate, or hydrate thereof.

5. Compound according to any one of claims 1, 3 or 4, wherein R¹⁰ is methyl, or a pharmacologically acceptable salt, solvate, or hydrate thereof.

6. Compound according to any one of claims 1 to 5, wherein R² is hydrogen, or a pharmacologically acceptable salt, solvate, or hydrate thereof.

7. Compound according to claim 1 or 2 having the following structure: 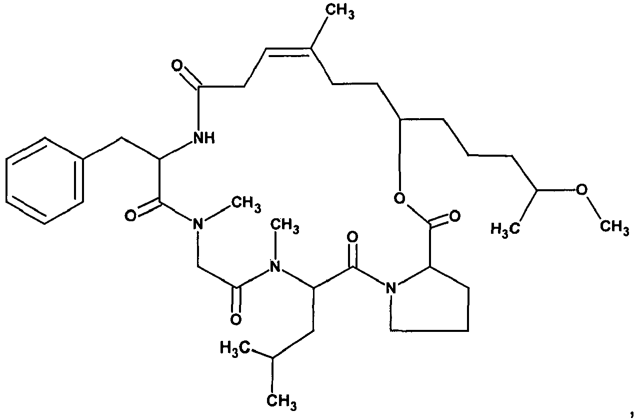 or a pharmacologically acceptable salt, solvate, or hydrate thereof.

8. Pharmaceutical composition comprising a compound according to any one of claims 1 to 7, or a pharmacologically acceptable salt, solvate, or hydrate thereof, and optionally one or more carrier substances and/or one or more adjuvants.

9. Compound or pharmaceutical composition according to any one of claims 1 to 8 for use in the treatment of viral infections.

10. A method of producing a compound according to any one of claims 1 to 7 **characterized in that** the *Byssovorax cruenta,* strain Har1 is used for the production.

## Patentansprüche

1. Verbindung der allgemeinen Formel (I): 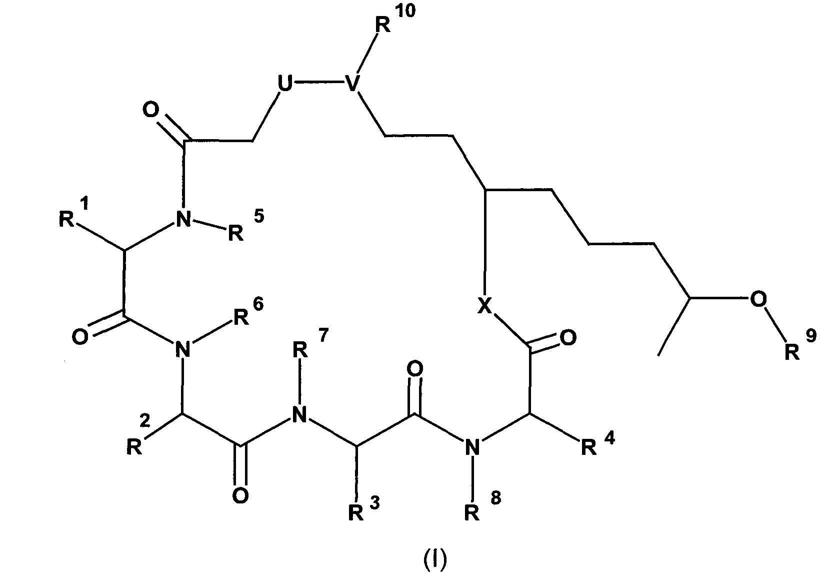 wobei
X gleich O oder NH ist;
U-V aus den folgenden Gruppen ausgewählt werden: 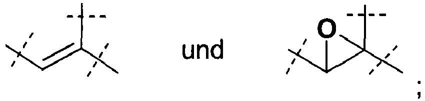
R¹ eine Gruppe der Formel -CH₂Ar ist, wobei Ar eine optional substituierte Aryl oder Heteroarylgruppe ist;
R² und R³ unabhängig aus H, CH₃, CH₂OH oder den folgenden Gruppen ausgewählt werden: 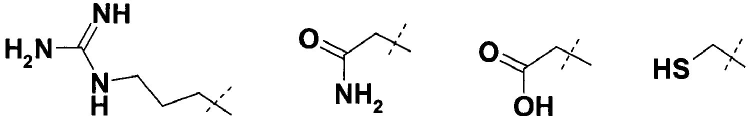 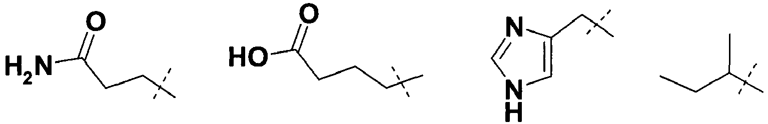 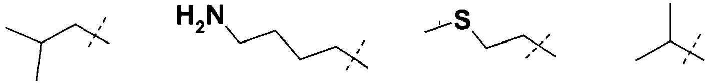 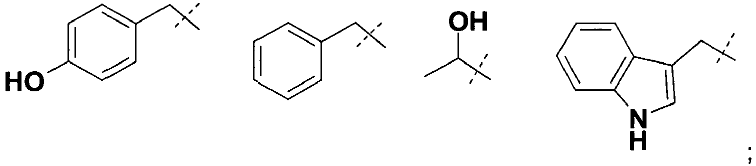
R⁴ und R⁸ zusammen eine Gruppe der Formel -(CH₂)ₙ- sind, wobei diese Gruppe optional substituiert sein kann, und n gleich 2, 3, 4 oder 5 ist;
R⁵, R⁶ und R⁷ unabhängig Wasserstoff oder C₁₋₄ Alkyl sind;
R⁹ ein Wasserstoffatom, eine Alkyl-, eine Alkenyl-, eine Alkinyl-, eine Heteroalkyl-, eine Cycloalkyl-, eine Heterocycloalkyl-, eine Alkylcycloalkyl-, eine Heteroalkylcycloalkyl-, eine Aryl-, eine Heteroaryl-, eine Aralkyl- oder eine Heteroaralkylgruppe ist; und
R¹⁰ gleich H oder methyl ist;
oder ein pharmakologisch akzeptables Salz, Solvat oder Hydrat davon;
wobei
sich der Ausdruck Alkyl auf eine gesättigte, geradkettige oder verzweigte Kohlenwasserstoffgruppe bezieht, die von 1 bis 6 Kohlenstoffatome enthält;
sich die Ausdrücke Alkenyl und Alkinyl auf zumindest teilweise ungesättigte, geradkettige oder verzweigte Kohlenwasserstoffgruppen beziehen, die von 2 bis 6 Kohlenstoffatome enthalten;
sich der Ausdruck Heteroalkyl auf eine Alkyl-, Alkenyl- oder Alkinylgruppe bezieht, bei der ein oder mehrere Kohlenstoffatom(e) durch ein Sauerstoff-, Stickstoff- oder Schwefelatom ersetzt wurde(n);
sich die Ausdrücke Alkyl, Alkenyl, Alkinyl und Heteroalkyl weiterhin auf Gruppen beziehen, bei denen ein oder mehrere Wasserstoffatom(e) durch ein Halogenatom ersetzt wurde(n);
sich der Ausdruck Cycloalkyl auf eine gesättigte oder teilweise ungesättigte cyclische Gruppe bezieht, die einen oder zwei Ringe enthält und von 3 bis 10 Kohlenstoffatome enthält; der Ausdruck Cycloalkyl bezieht sich weiterhin auf Gruppen, bei denen ein oder mehrere Wasserstoffatom(e) durch Fluor-, Chlor-, Brom- oder Jodatome oder durch OH, =O, SH, =S, NH₂, =NH oder NO₂ Gruppen ersetzt wurde(n);
sich der Ausdruck Heterocycloalkyl auf eine Cycloalkylgruppe bezieht, wie sie oben definiert ist, bei der ein oder mehrere Ring-Kohlenstoffatom(e) durch ein Sauerstoff-, Stickstoff- oder Schwefelatom ersetzt wurde(n);
sich der Ausdruck Alkylcycloalkyl auf Gruppen bezieht, die sowohl Cycloalkyl-als auch Alkyl-, Alkenyl- oder Alkinylgruppen in Übereinstimmung mit den oben angegebenen Definitionen enthalten;
sich der Ausdruck Heteroalkylcycloalkyl auf Alkylcycloalkylgruppen bezieht, wie sie oben definiert sind, bei denen ein oder mehrere Kohlenstoffatom(e) durch ein Sauerstoff-, Stickstoff- oder Schwefelatom ersetzt wurde(n);
sich der Ausdruck Aryl auf eine aromatische Gruppe bezieht, die einen oder mehrere Ringe aufweist und von 6 bis 10 Kohlenstoffatome enthält; der Ausdruck Aryl bezieht sich weiterhin auf Gruppen, bei denen ein oder mehrere Wasserstoffatom(e) durch Fluor-, Chlor-, Brom- oder Jodatome oder durch OH, SH, NH₂ oder NO₂ Gruppen ersetzt wurde(n);
sich der Ausdruck Heteroaryl auf eine aromatische Gruppe bezieht, die einen oder mehrere Ringe aufweist und von 5 bis 10 Ringatome enthält und die ein oder mehrere Sauerstoff-, Stickstoff- oder Schwefelatom(e) enthält; der Ausdruck Heteroaryl bezieht sich weiterhin auf Gruppen, bei denen ein oder mehrere Wasserstoffatom(e) durch Fluor-, Chlor-, Brom- oder Jodatome oder durch OH, SH, NH₂ oder NO₂ Gruppen ersetzt wurde(n);
sich der Ausdruck Aralkyl auf Gruppen bezieht, die sowohl Aryl- als auch Alkyl-, Alkenyl-, Alkinyl- und/oder Cycloalkylgruppen in Übereinstimmung mit den oben angegebenen Definitionen enthalten;
sich der Ausdruck Heteroaralkyl auf Aralkylgruppen bezieht, wie sie oben definiert sind, bei denen ein oder mehrere Kohlenstoffatom(e) durch ein Sauerstoff-, Stickstoff- oder Schwefelatom ersetzt wurde(n); und
sich der Ausdruck "optional substituiert" auf Gruppen bezieht, bei denen ein oder mehrere Wasserstoffatom(e) optional durch Fluor-, Chlor-, Brom- oder Jodatome oder durch OH, =O, SH, =S, NH₂, =NH oder NO₂ Gruppen ersetzt wurde(n) und wobei sich dieser Ausdruck weiterhin auf Gruppen bezieht, die optional durch eine oder mehrere unsubstituierte C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkylgruppe(n) substituiert sind.

2. Verbindung nach Anspruch 1, welche die folgende allgemeine Formel (II) aufweist: 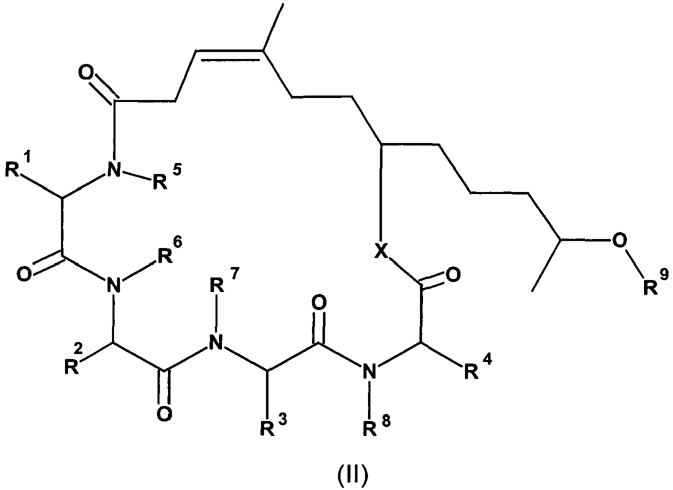 wobei R¹, R², R³, R⁴, R⁵, R⁶, R⁷, R⁸, R⁹ und X wie in Anspruch 1 definiert sind, oder ein pharmakologisch akzeptables Salz, Solvat oder Hydrat davon.

3. Verbindung nach Anspruch 1 or 2, wobei X gleich O ist, oder ein pharmakologisch akzeptables Salz, Solvat oder Hydrat davon.

4. Verbindung nach einem der Ansprüche 1 bis 3, wobei R⁹ methyl ist, oder ein pharmakologisch akzeptables Salz, Solvat oder Hydrat davon.

5. Verbindung nach einem der Ansprüche 1, 3 oder 4, wobei R¹⁰ methyl ist, oder ein pharmakologisch akzeptables Salz, Solvat oder Hydrat davon.

6. Verbindung nach einem der Ansprüche 1 bis 5, wobei R² Wasserstoff ist, oder ein pharmakologisch akzeptables Salz, Solvat oder Hydrat davon.

7. Verbindung nach Anspruch 1 oder 2, welche die folgende Struktur aufweist: 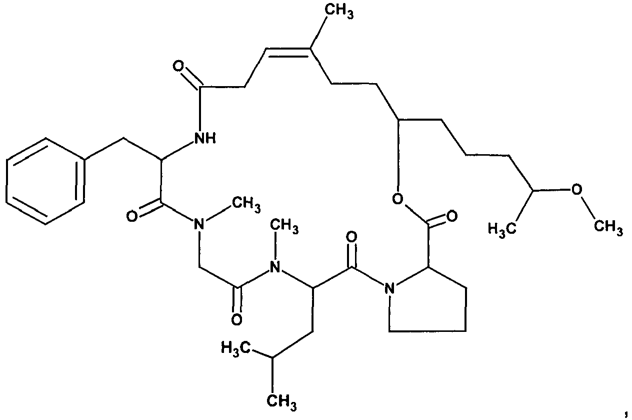 oder ein pharmakologisch akzeptables Salz, Solvat oder Hydrat davon.

8. Pharmazeutische Zusammensetzung umfassend eine Verbindung nach einem der Ansprüche 1 bis 7, oder ein pharmakologisch akzeptables Salz, Solvat oder Hydrat davon, und optional einen oder mehrere Trägerstoff(e) und/oder einen oder mehrere Hilfsmittel.

9. Verbindung oder Pharmazeutische Zusammensetzung nach einem der Ansprüche 1 bis 8 zur Verwendung bei der Behandlung von Virusinfektionen.

10. Verfahren zur Herstellung einer Verbindung nach einem der Ansprüche 1 bis 7, **dadurch gekennzeichnet, dass** der *Byssovorax cruenta* Stamm Har1 zur Herstellung verwendet wird.

## Revendications

1. Composé de formule générale (I) : 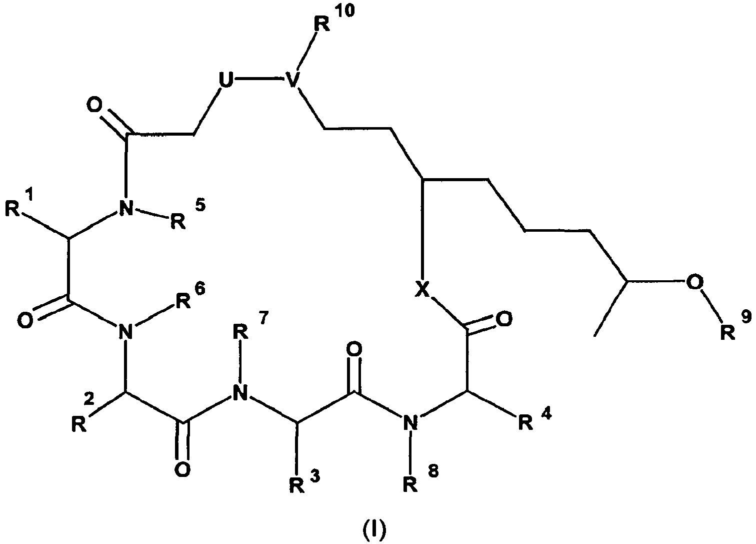 dans laquelle
X représente O ou NH ;
U-V sont choisis parmi les groupes suivants : 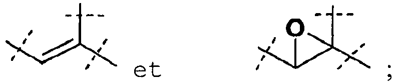
R¹ est un groupe de formule -CH₂Ar dans lequel Ar représente un groupe aryle ou hétéroaryle éventuellement substitué ;
R² et R³ sont choisis indépendamment parmi H, CH₃, CH₂OH ou les groupes suivants : 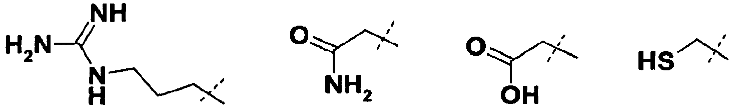 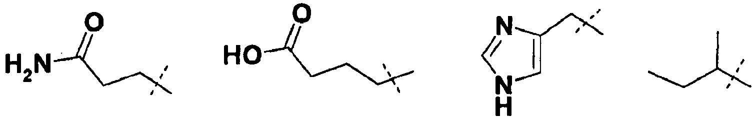 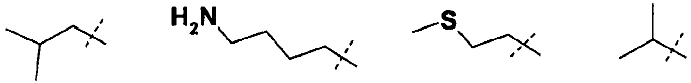 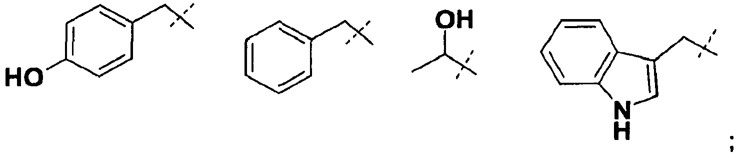
R⁴ et R⁸ ensemble représentent un groupe de formule -(CH₂)ₙ-, ce groupe pouvant être éventuellement substitué et n valant 2, 3, 4 ou 5 ;
R⁵, R⁶ et R⁷ représentent indépendamment un atome d'hydrogène ou un groupe alkyle en C₁ à C₄ ;
R⁹ représente un atome d'hydrogène, un groupe alkyle, alcényle, alcynyle, hétéroalkyle, cycloalkyle, hétérocycloalkyle, alkylcycloalkyle, hétéroalkylcycloalkyle, aryle, hétéroaryle, aralkyle ou hétéroaralkyle ; et
R¹⁰ représente H ou un groupe méthyle ;
ou l'un de ses sels pharmacologiquement acceptables, solvates, ou hydrates ;
dans lequel
l'expression alkyle se rapporte à un groupe hydrocarbure saturé à chaîne linéaire ou ramifiée qui contient de 1 à 6 atomes de carbone ;
les expressions alcényle et alcynyle se rapportent à des groupes hydrocarbures au moins partiellement insaturés à chaîne linéaire ou ramifiée qui contiennent de 2 à 6 atomes de carbone ;
l'expression hétéroalkyle se rapporte à un groupe alkyle, alcényle ou alcynyle dans lequel un ou plusieurs atomes de carbone ont été remplacés par un atome d'oxygène, d'azote ou de soufre ;
les termes alkyle, alcényle, alcynyle et hétéroalkyle se rapportent en outre à des groupes dans lesquels un ou plusieurs atomes d'hydrogène ont été remplacés par un atome d'halogène ;
l'expression cycloalkyle se rapporte à un groupe cyclique saturé ou partiellement insaturé qui contient un ou deux cycles, contenant de 3 à 10 atomes de carbone ; l'expression cycloalkyle se rapporte en outre à des groupes dans lesquels un ou plusieurs atomes d'hydrogène ont été remplacés par des atomes de fluor, de chlore, de brome ou d'iode ou par des groupes OH, =0, SH, =S, NH₂, =NH ou NO₂ ;
l'expression hétérocycloalkyle se rapporte à un groupe cycloalkyle tel que défini ci-dessus dans lequel un ou plusieurs atomes de carbone du cycle ont été remplacés par un atome d'oxygène, d'azote ou de soufre ;
l'expression alkylcycloalkyle se rapporte à des groupes contenant des groupes à la fois cycloalkyle et également alkyle, alcényle ou alcynyle conformément aux définitions ci-dessus ;
l'expression hétéroalkylcycloalkyle se rapporte à des groupes alkylcycloalkyle tels que définis ci-dessus dans lesquels un ou plusieurs atomes de carbone ont été remplacés par un atome d'oxygène, d'azote ou de soufre ;
l'expression aryle se rapporte à un groupe aromatique qui comporte un ou plusieurs cycles contenant de 6 à 10 atomes de carbone ; l'expression aryle se rapporte en outre à des groupes dans lesquels un ou plusieurs atomes d'hydrogène ont été remplacés par des atomes de fluor, de chlore, de brome ou d'iode ou par des groupes OH, SH, NH₂ ou NO₂ ;
l'expression hétéroaryle se rapporte à un groupe aromatique qui comporte un ou plusieurs cycles contenant de 5 à 10 atomes de cycle, et contient un ou plusieurs atomes d'oxygène, d'azote ou de soufre de cycle ; l'expression hétéroaryle se rapporte en outre à des groupes dans lesquels un ou plusieurs atomes d'hydrogène ont été remplacés par des atomes de fluor, de chlore, de brome ou d'iode ou par des groupes OH, SH, NH₂ ou NO₂ ;
l'expression aralkyle se rapporte à des groupes contenant des groupes à la fois aryle et également alkyle, alcényle, alcynyle et/ou cycloalkyle conformément aux définitions ci-dessus ;
l'expression hétéroaralkyle se rapporte à un groupe aralkyle tel que définis ci-dessus dans lequel un ou plusieurs atomes de carbone ont été remplacés par un atome d'oxygène, d'azote ou de soufre ; et
l'expression « éventuellement substitué » se rapporte à des groupes dans lesquels un ou plusieurs atomes d'hydrogène ont été éventuellement remplacés par des atomes de fluor, de chlore, de brome ou d'iode ou par des groupes OH, =O, SH, =S, NH₂, =NH ou NO₂ et cette expression se rapportant en outre à des groupes qui sont éventuellement substitués par un ou plusieurs groupes non substitués alkyle en C₁ à C₆, alcényle en C₂ à C₆, alcynyle en C₂ à C₆, hétéroalkyle en C₁ à C₆, cycloalkyle en C₃ à C₁₀, hétérocycloalkyle en C₂ à C₉, aryle en C₆ à C₁₀, hétéroaryle en C₁ à C₉, aralkyle en C₇ à C₁₂ ou hétéroaralkyle en C₂ à C₁₁.

2. Composé selon la revendication 1, ayant la formule générale (II) suivante : 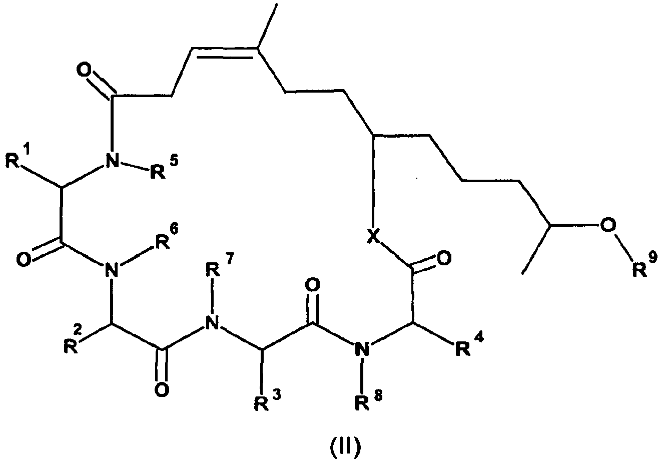 dans laquelle R¹, R², R³, R⁴, R⁵, R⁶, R⁷, R⁸, R⁹ et X sont définis comme dans la revendication 1, ou l'un de ses sels pharmacologiquement acceptables, solvates, ou hydrates.

3. Composé selon la revendication 1 ou 2, dans lequel X représente 0, ou l'un de ses sels pharmacologiquement acceptables, solvates, ou hydrates.

4. Composé selon l'une quelconque des revendications 1 à 3, dans lequel R⁹ représente un groupe méthyle, ou l'un de ses sels pharmacologiquement acceptables, solvates, ou hydrates.

5. Composé selon l'une quelconque des revendications 1, 3 ou 4, dans lequel R¹⁰ représente un groupe méthyle, ou l'un de ses sels pharmacologiquement acceptables, solvates, ou hydrates.

6. Composé selon l'une quelconque des revendications 1 à 5, dans lequel R² représente un atome d'hydrogène, ou l'un de ses sels pharmacologiquement acceptables, solvates, ou hydrates.

7. Composé selon la revendication 1 ou 2 ayant la structure suivante : 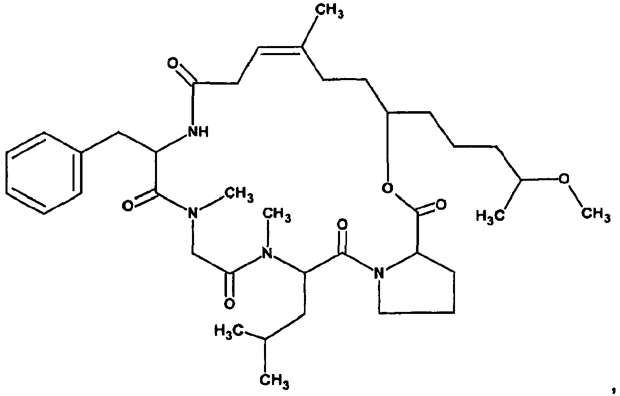 ou l'un de ses sels pharmacologiquement acceptables, solvates, ou hydrates.

8. Composition pharmaceutique comprenant un composé selon l'une quelconque des revendications 1 à 7, ou l'un de ses sels pharmacologiquement acceptables, solvates, ou hydrates, et éventuellement une ou plusieurs substances de support et/ou un ou plusieurs adjuvants.

9. Composé ou composition pharmaceutique selon l'une quelconque des revendications 1 à 8 pour une utilisation dans le traitement d'infections virales.

10. Procédé de production d'un composé selon l'une quelconque des revendications 1 à 7 **caractérisé en ce que** la souche Har1 de *Byssovorax cruenta* est utilisée pour la production.
